Europäisches Patentamt

(19) European Patent Office

Office européen des brevets

(11) Veröffentlichungsnummer: **0 153 475**
A2

## (12) EUROPÄISCHE PATENTANMELDUNG

(21) Anmeldenummer: 84115507.0

(51) Int. Cl.⁴: **A 61 K 6/08**

(22) Anmeldetag: 15.12.84

(30) Priorität: 03.02.84 DE 3403777

(43) Veröffentlichungstag der Anmeldung:
04.09.85 Patentblatt 85/36

(84) Benannte Vertragsstaaten:
AT BE CH DE FR GB IT LI NL SE

(71) Anmelder: Degussa Aktiengesellschaft
Weissfrauenstrasse 9
D-6000 Frankfurt am Main 1(DE)

(72) Erfinder: Beyer, Hans-Hermann, Dr. Dipl.-Chem.
Am Birkenanger 11
D-8756 Kahl(DE)

(72) Erfinder: Diehl, Walter, Dr. Dipl.-Phys.
Gustav-Adolf-Strasse 29
D-6450 Hanau 1(DE)

(72) Erfinder: Eckert, Karlheinz, Dipl.-Ing.
Wibaustrasse 2
D-6466 Gründau 2(DE)

(72) Erfinder: Eiermann, Kurt, Dr. Dipl.-Phys.
Friedhofstrasse 26
D-6102 Pfungstadt 2(DE)

(72) Erfinder: Ringelstein, Hans-Martin
Inheidenerstrasse 22
D-6000 Frankfurt 60(DE)

(54) Verfahren zur Herstellung von Zahnfüllungen.

(57) Zur Herstellung von Zahnfüllungen werden Pulver aus duktilen, mundbeständigen Metallen, wie Silber, Platin, Palladium, Aluminium und Titan, bzw. deren Legierungen oder aus kaltverschweißbaren, mundbeständigen Kunststoffen, wie Polyacrylate, Polycarbonate und Polysulfone, mit einem bei 20 bis 45° C sich verflüssigenden, plastischen Bindemittel zu einer Paste verarbeitet. Diese Paste wird in der Zahnkavität unter Einwirkung von Ultraschall verfestigt.

EP 0 153 475 A2

0153475

84 107 DT

Degussa Aktiengesellschaft

Weissfrauenstraße 9, 6000 Frankfurt/Main

## Verfahren zur Herstellung von Zahnfüllungen

Die Erfindung betrifft ein Verfahren zur Herstellung von Zahnfüllungen durch Einbringung eines pulverförmigen Materials in die Kavität mit anschließender mechanischer Verfestigung.

In der konservierenden Zahnheilkunde sind eine Reihe von metallischen Füllstoffen bekannt, wie beispielsweise Amalgame, Gußlegierungen in Form von Inlays oder Stopfgold. Die Füllung der Zahnkavitäten mit Stopfgold ist eine der ältesten Zahnfüllungsmethoden. Für diese Goldstopffüllungen wird chemisch reines Gold in Form von Goldfolie, Goldschwamm oder Goldpulver verwendet.

Die aus reinem Gold hergestellten Goldstopffüllungen werden in Bezug auf Haltbarkeit, Aestethik und Korrosionsbeständigkeit ausgezeichnet beurteilt. Schwerwiegende Nachteile der Goldstopffüllungen sind jedoch das technisch und zeitlich sehr aufwendige Präparieren der Kavität und das ebenfalls großes Geschick erfordernde Legen der Füllung. So ist zunächst eine sehr sorgfältige Bearbeitung der Kavität mit Unterschnitten und eine nicht automatisch durchführbare Aufrauhung der Kavitätenwände nötig. Beides ist unbedingte Voraussetzung für eine ausreichende Haftung des Goldes in der Kavität. Desweiteren muß die Kavität während des Goldstopfvorganges absolut frei von Feuchtigkeit sein. Dies betrifft nicht nur den Speichelfluß, sondern auch die Atemluft des Patienten. Dies macht die ebenfalls zeitraubende und für den Patienten mitunter sehr unangenehme Anwendung von sogenannten Cofferdam-Folien erforderlich.

Darüberhinaus muß das Material der Goldstopffüllung unmittelbar vor dem Einbringen in die Kavität in einer sehr sauberen Alkoholflamme ausgeglüht werden, damit sämtliche Verunreinigungen auf der Oberfläche entfernt werden und eine kohäsive Bindung zwischen den einzelnen Goldteilchen erreicht wird. Die Kaltschweißbarkeit des Goldes, die Grundlage des Goldstopfens, wird durch eine Kontamination der Oberfläche, insbesondere durch Flüssigkeitsfilme, sehr stark vermindert.

Aus der DE-OS 30 42 008 ist ein Verfahren bekannt, bei dem poröse Sinterkörper oder Drahtgeflechtknäuel aus Gold-, Silber-, Platin-, Eisen-, Nickel-, Kobalt-, Aluminium- und Titanlegierungen zusammen mit einem plastischen oder flüssigen organischen Bindemittel in die Kavität eingebracht und mit manuellen Stopfgeräten der Kavität angepasst und verfestigt werden. Mit diesem Verfahren lassen sich allerdings keine einwandfreien Oberflächen erzeugen.

Es war daher Aufgabe der vorliegenden Erfindung, ein Verfahren zur Herstellung von Zahnfüllungen zu entwickeln, durch Einbringung eines pulverförmigen Materials in die Kavität und anschließender mechanischer Verfestigung, das nicht empfindlich auf Feuchtigkeit ist, ein rasches Arbeiten erlaubt und einwandfreie Oberflächen liefert.

Diese Aufgabe wurde erfindungsgemäß dadurch gelöst, daß Pulver aus duktilen, mundbeständigen Metallen bzw. Legierungen oder aus kaltverschweißbaren, mundbeständigen Kunststoffen verwendet werden, die mit einem bei 20 bis 45$^{\circ}$ C sich verflüssigenden, plastischen organischen Bindemittel zu einer Paste verarbeitet werden, und daß die mechanische Verfestigung unter Einwirkung von Ultraschall erfolgt.

Vorzugsweise verwendet man als Bindemittel Polyäthylenglycol mit einem Molekulargewicht von 600 bis 1500 in Mengen von 0,5 bis 5 Gew%. Als sehr vorteilhaft hat sich ein Zusatz von rund 2,6 Gew% Polyäthylenglycol erwiesen. Das Bindemittel muß physiologisch unbedenklich und sollte vorteilhafterweise wasserlöslich bzw. speichellöslich sein.

Als Metallpulver haben sich vor allem Silber, Platin, Palladium, Aluminium und Titan bzw. deren Legierungen als geeignet erwiesen, als Kunststoffpulver Polyacrylate (insbesondere Polymethacrylate),Polycarbonate, Polysulfone und isotaktisches Polypropylen.

Aus den Pulvern und dem Bindemittel wird eine Paste hergestellt und diese mit einem geeigneten Werkzeug portionsweise in die Zahnkavität eingebracht, wo sie mit einer Sonotrode unter Druck- und Schalleinwirkung verfestigt wird. Als Anpreßkraft ist beispielsweise eine Kraft von 6 N erforderlich, bei einer Ultraschallfrequenz von 28 Kilohertz.

Degussa Aktiengesellschaft,

Weissfrauenstraße 9, 6000 Frankfurt/Main

Patentansprüche:

1. Verfahren zur Herstellung von Zahnfüllungen durch Einbringung eines pulverförmigen Materials in die Kavität mit anschließender mechanischer Verfestigung,

   dadurch gekennzeichnet, daß Pulver aus duktilen, mundbeständigen Metallen bzw. Legierungen oder aus kaltverschweißbaren, mundbeständigen Kunststoffen verwendet werden, die mit einem bei 20 bis 45° C sich verflüssigenden, plastischen Bindemittel zu einer Paste verarbeitet werden, und daß die mechanische Verfestigung unter Einwirkung von Ultraschall erfolgt.

2. Verfahren zur Herstellung von Zahnfüllungen nach Anspruch 1,

   dadurch gekennzeichnet, daß als Bindemittel Polyäthylenglycol mit einem Molekulargewicht von 600 bis 1500 in Mengen von 0,5 bis 5 Gew% verwendet wird.

- 5 -

0153475

3. Verfahren zur Herstellung von Zahnfüllungen
nach Anspruch 1 und 2,
dadurch gekennzeichnet,
daß als Metallpulver Silber, Platin, Palladium,
Aluminium oder Titan, bzw. deren Legierungen als
Kunststoffpulver Polyacrylate, Polycarbonate, isotaktisches Polypropylen oder Polysulfone verwendet
werden.